(19) **Europäisches Patentamt**
**European Patent Office**
**Office européen des brevets**

(11) **EP 3 488 261 B1**

(12) **FASCICULE DE BREVET EUROPEEN**

(45) Date de publication et mention de la délivrance du brevet:
**12.08.2020 Bulletin 2020/33**

(21) Numéro de dépôt: **17748529.9**

(22) Date de dépôt: **10.07.2017**

(51) Int Cl.:
*G01S 15/36* $^{(2006.01)}$ *A61B 5/113* $^{(2006.01)}$
*G01S 15/50* $^{(2006.01)}$

(86) Numéro de dépôt international:
**PCT/FR2017/051883**

(87) Numéro de publication internationale:
**WO 2018/015638 (25.01.2018 Gazette 2018/04)**

(54) **PROCÉDÉ ET DISPOSITIF ACOUSTIQUE POUR MESURER DES MOUVEMENTS DE SURFACE**

AKUSTISCHES VERFAHREN UND AKUSTISCHE VORRICHTUNG ZUR MESSUNG VON OBERFLÄCHENBEWEGUNGEN

ACOUSTIC METHOD AND DEVICE FOR MEASURING SURFACE MOVEMENTS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **21.07.2016 FR 1656935**

(43) Date de publication de la demande:
**29.05.2019 Bulletin 2019/22**

(73) Titulaires:
- **Centre National de la Recherche Scientifique CNRS**
  **75016 Paris (FR)**
- **Ecole Supérieure de Physique et de Chimie Industrielles de la Ville de Paris**
  **75005 Paris (FR)**
- **Sorbonne Université**
  **75006 Paris (FR)**
- **Institut National de la Santé et de la Recherche Médicale (INSERM)**
  **75013 Paris (FR)**
- **Université Paris Diderot - Paris 7**
  **75013 Paris (FR)**

(72) Inventeurs:
- **ING, Ros Kiri**
  **94200 Ivry Sur Seine (FR)**
- **JEGER-MADIOT, Nathan**
  **75013 Paris (FR)**
- **FINK, Mathias**
  **92190 Meudon (FR)**
- **SIMILOWSKI, Thomas**
  **92130 Issy Les Moulineaux (FR)**

(74) Mandataire: **Osha Liang**
**2, rue de la Paix**
**75002 Paris (FR)**

(56) Documents cités:
**US-A- 4 122 427        US-A- 4 197 856**
**US-A1- 2011 208 060     US-A1- 2015 078 131**

- **CATHELINE STEFAN ET AL: "Acoustic source localization model using in-skull reverberation and time reversal", APPLIED PHYSICS LETTERS, A I P PUBLISHING LLC, US, vol. 90, no. 6, 7 février 2007 (2007-02-07), pages 63902-063902, XP012095996, ISSN: 0003-6951, DOI: 10.1063/1.2431470 cité dans la demande**
- **ETAIX NICOLAS ET AL: "Acoustic imaging device with one transducer", THE JOURNAL OF THE ACOUSTICAL SOCIETY OF AMERICA, AMERICAN INSTITUTE OF PHYSICS FOR THE ACOUSTICAL SOCIETY OF AMERICA, NEW YORK, NY, US, vol. 131, no. 5, 1 mai 2012 (2012-05-01), pages EL395-EL399, XP012160225, ISSN: 0001-4966, DOI: 10.1121/1.3699533 [extrait le 2012-04-13] cité dans la demande**
- **NICOLAS ETAIX ET AL: "Increasing the modal density in plates for mono-element focusing in air", THE JOURNAL OF THE ACOUSTICAL SOCIETY OF AMERICA, vol. 134, no. 2, 1 août 2013 (2013-08-01), pages 1049-1054, XP055356031, New York, NY, US ISSN: 0001-4966, DOI: 10.1121/1.4812260 cité dans la demande**

**EP 3 488 261 B1**

- QUIEFFIN N ET AL: "2D pseudo-array using an ultrasonic one channel time-reversal mirror", 2004 IEEE ULTRASONICS SYMPOSIUM : 23 - 27 AUGUST 2004, PALAIS DES CONGR GBP ES, MONTRÉAL, CANADA, IEEE OPERATIONS CENTER, PISCATAWAY, NJ, vol. 1, 23 août 2004 (2004-08-23), pages 801-804, XP010786596, DOI: 10.1109/ULTSYM.2004.1417843 ISBN: 978-0-7803-8412-5

**Description**

DOMAINE DE L'INVENTION

La présente invention est relative aux procédés et dispositifs acoustiques pour détecter des mouvements de surface.

Plus particulièrement, l'invention concerne un procédé pour détecter des mouvements d'une surface, comprenant une étape de mesure au cours de laquelle on émet au moins une onde ultrasonore incidente dans l'air vers la surface avec un dispositif d'émission d'ondes ultrasonores et on capte des signaux réfléchis représentatifs d'au moins une onde ultrasonore réfléchie dans l'air par ladite surface à partir de ladite au moins une onde ultrasonore incidente.

Le document US4122427 décrit un exemple d'un tel procédé, dans lequel on mesure les mouvements d'une surface sur une voie de mesure, en faisant émettre vers la surface, des ultrasons à fréquence de l'ordre de 40 Hz. Les ultrasons sont émis par un transducteur unique.

Le document US2011208060 décrit également un exemple d'un tel procédé, dans lequel on mesure les mouvements d'une surface sur une ou plusieurs zones distinctes de mesure, en faisant émettre vers la surface des ondes ultrasonores générées au moyen d'un transducteur unique ou au moyen d'un réseau de transducteurs.

RESUME DE L'INVENTION

La présente invention a notamment pour but de perfectionner encore ce type de procédé, notamment afin de permettre une meilleure efficacité de détection des mouvements de la surface.

A cet effet, selon l'invention, un procédé du genre en question est caractérisé en ce qu'au cours de chaque de mesure :

- on mesure les mouvements d'une pluralité de points de mesure appartenant au moins à ladite surface en illuminant chaque point de mesure par ladite au moins une onde ultrasonore incidente sous une multiplicité d'angles d'incidence,
- on capte les signaux réfléchis avec un réseau de transducteurs de réception comprenant une pluralité de transducteurs ultrasonores de réception et on détermine un signal de formation de voie pour chaque point de mesure, par formation de voie au moins en réception à partir desdits signaux réfléchis,

et en ce qu'il comporte en outre au moins une étape de détermination de mouvement au cours de laquelle on détermine lesdits mouvements de la surface au point de mesure considéré en déterminant au moins un retard ou un déphasage entre deux signaux de formation de voie pour ce point de mesure.

Grâce à ces dispositions, on peut mesurer les mouvements d'une grande variété de surfaces liquides ou solides, lisses ou rugueuses, planes ou non, et ce indépendamment de leur transparence à la lumière. On peut imager les mouvements de la surface sur une aire importante, par exemple plusieurs dizaines de $cm^2$, à une cadence pouvant atteindre le kilohertz voire plus. La sensibilité du procédé de mesure de l'invention atteint le micromètre pour une vitesse minimale détectable de l'ordre de la fraction du millimètre par seconde. Enfin la puissance acoustique mise en œuvre peut être faible, par exemple avec un niveau de l'ordre de 60-70 dB SPL (niveau de pression acoustique).

Dans des modes de réalisation préférés du procédé selon l'invention, on peut éventuellement avoir recours en outre à l'une et/ou à l'autre des dispositions suivantes :

- au cours de chaque étape de mesure, on illumine chaque point de mesure de la surface avec ladite au moins une onde incidente sous des angles d'incidence s'étendant sur une plage d'angles d'incidence d'au moins à 20 degrés ;
- au cours de chaque étape de mesure, on mesure les mouvements en sensiblement tout point de la surface sur une aire supérieure à 10 $cm^2$ ;
- le dispositif d'émission d'ondes ultrasonores et le réseau de transducteurs de réception sont bidimensionnels ;
- le réseau de transducteurs d'émission (2) présente une ouverture au moins égale à une ouverture du dispositif de réception d'ondes ultrasonores selon deux directions sensiblement perpendiculaires ;
- l'ouverture du réseau de transducteurs d'émission est au moins égale à trois fois l'ouverture du dispositif de réception d'ondes ultrasonores, selon au moins une des directions ;
- l'ouverture du dispositif d'émission d'ondes ultrasonores est au moins égale à 20 cm dans chaque direction ;
- le dispositif d'émission d'ondes ultrasonores comprend un réseau de transducteurs d'émission comprenant une pluralité de transducteurs ultrasonores d'émission ;
- les transducteurs ultrasonores d'émission sont répartis en plusieurs groupes et au cours de ladite étape de mesure, on fait émettre simultanément un même signal par les transducteurs ultrasonores d'émission appartenant à un même groupe ;
- le dispositif d'émission d'ondes ultrasonores comprend au moins un transducteur ultrasonore d'émission disposé pour émettre dans une cavité mélangeuse adaptée pour provoquer des réflexions multiples de ladite au moins une onde ultrasonore incidente avant envoi vers la surface ;
- les ondes ultrasonores ont une fréquence inférieure à 100 kHz ;
- les ondes ultrasonores sont émises à une cadence supérieure à 500 tirs par seconde ;
- au cours de chaque étape de mesure d'indice k, on calcule au moins en différents points de la surface un signal de formation de voie $S_k$ en réception audit point, et au cours de chaque étape de détermination de mouvement, on détermine le mouvement de chaque point de la surface en déterminant un retard ou un déphasage entre les signaux formation de voie $S_k$ en réception audit point, pour deux valeurs de k différentes.
- au cours de chaque étape de mesure d'indice k, le dispositif d'émission d'ondes ultrasonores émet une onde ultrasonore incidente non focalisée vers la surface ;
- au cours de chaque étape de mesure d'indice k, le dispositif d'émission d'ondes ultrasonores émet successivement des ondes ultrasonore incidente focalisées vers les différents points de la surface et le signal de formation de voie $S_k$ en réception audit point est déterminé à partir des signaux réfléchis correspondant à l'onde ultrasonore incidente focalisée audit point ;
- le signal de formation de voie en réception $S_k$ est déterminé par la formule :

$$S_k(t) = \sum_{j=1}^{N} r_j\left(t - \frac{d_j}{C}\right)$$

où :

- $r_j$ est le signal capté par le transducteur ultrasonore de réception (3a) d'indice j,
- t est le temps,
- $d_j$ est une distance entre le point P et le transducteur ultrasonore de réception (3a) d'indice j,
- c est la célérité de l'onde ultrasonore dans l'air.

- le dispositif d'émission d'ondes ultrasonores comprend un réseau de transducteurs d'émission comprenant une pluralité de transducteurs ultrasonores d'émission, au cours de chaque étape de mesure d'indice k, on mesure des réponses impulsionnelles respectives entre chaque transducteur ultrasonore d'émission et chaque transducteur ultrasonore de réception, puis on calcule au moins en différents points de la surface un signal de formation de voie $S'_k$ en émission et réception audit point,
et au cours de chaque étape de détermination de mouvement, on détermine un mouvement de chaque point de la surface en déterminant un retard ou un déphasage entre les signaux de voie $S'_k$ en émission et réception audit point, pour deux valeurs de k différentes ;
- chaque signal de formation de voie en émission et réception audit point est déterminé par la formule :

$$S'_k(t) = \sum_{j=1}^{N} \sum_{i=1}^{M} h_{ijk}\left(t - \frac{d_{ij}}{c}\right) \ ,$$

où :

- i est un indice compris entre 1 et M désignant un transducteur ultrasonore d'émission,
- j est un indice compris entre 1 et N désignant un transducteur ultrasonore de réception,
- $h_{ijk}$ est la réponse impulsionnelle entre le transducteur ultrasonore d'émission d'indice i et le transducteur ultrasonore de réception d'indice j,
- t est le temps,
- $d_{ij}$ est une distance parcourue par une onde ultrasonore depuis le transducteur ultrasonore d'émission d'indice i jusqu'au transducteur ultrasonore de réception d'indice j en se réfléchissant au point (P) considéré de la surface,
- c est la célérité des ondes ultrasonores dans l'air ;

- au cours d'au moins certaines étapes de mesure, on détermine un signal de formation de voie pour chaque point de mesure dans une zone d'observation prédéterminée et on détermine les points de mesure appartenant à la surface comme étant ceux qui maximisent le signal de formation de voie ;
- au cours de l'étape de détermination de mouvement, on détermine un retard dt entre des signaux de formation de voie correspondant à deux étapes de mesures au même point de mesure et on détermine :

- un déplacement δ au point de mesure comme étant proportionnel dt.c
- et / ou une vitesse au point de mesure comme étant proportionnelle à dt.c/$\Delta$t,
  où c est la célérité des ondes ultrasonores dans l'air et $\Delta$t est un intervalle de temps entre lesdites deux étapes de mesure ;
- au cours de l'étape de détermination de mouvement, on détermine un déphasage $\varphi$ entre des signaux de formation de voie correspondant à deux étapes de mesures au même point de mesure et on détermine :
- un déplacement δ au point de mesure comme étant proportionnel c.$\varphi$/(2.$\pi$.f)
- et / ou une vitesse au point de mesure comme étant proportionnelle à c.$\varphi$/(2.$\pi$.f)/$\Delta$t,
  où c est la célérité des ondes ultrasonores dans l'air, f la fréquence des ondes ultrasonores et $\Delta$t est un intervalle de temps entre lesdites deux étapes de mesure.

Par ailleurs, l'invention a également pour objet un dispositif pour détecter des mouvements d'une surface réfléchissant les ondes ultrasonores, comprenant un dispositif d'émission d'ondes ultrasonores, un dispositif de réception d'ondes ultrasonores un dispositif de commande commandant le dispositif d'émission d'ondes ultrasonores et recevant des signaux captés par le dispositif de réception d'ondes ultrasonores, le dispositif de commande étant adapté pour réaliser plusieurs étapes de mesure successives au cours de chacune desquelles le dispositif d'émission d'ondes ultrasonores émet au moins une onde ultrasonore incidente dans l'air vers la surface et le dispositif de réception d'ondes ultrasonores capte des signaux réfléchis représentatifs d'au moins une onde ultrasonore réfléchie dans l'air par ladite surface à partir de ladite au moins une onde ultrasonore incidente,
caractérisé en ce que dispositif d'émission d'ondes ultrasonores est adapté pour illuminer une pluralité de point de mesure (P) appartenant au moins à ladite surface (21) par ladite au moins une onde ultrasonore incidente sous une multiplicité d'angles d'incidence,
en ce que le dispositif de réception d'ondes ultrasonores est un réseau de transducteurs de réception comprenant une pluralité de transducteurs ultrasonores de réception,
en ce que le dispositif de commande est adapté pour, au cours de chaque étape de mesure, déterminer un signal de formation de voie pour chaque point de mesure, par formation de voie au moins en réception à partir desdits signaux réfléchis,
et en ce que le dispositif de commande est adapté pour déterminer lesdits mouvements de la surface au point de mesure considéré en déterminant au moins un retard ou un déphasage entre deux signaux de formation de voie pour ce point de mesure.

D'autres caractéristiques et avantages de l'invention apparaîtront au cours de la description suivante d'une de ses formes de réalisation, donnée à titre d'exemple non limitatif, en regard des dessins joints.

Sur les dessins :

- la figure 1 est une vue d'ensemble schématique d'un exemple de dispositif de génération d'ondes ultrasonores mettant en œuvre l'invention,
- la figure 2 montre un exemple de réseau de transducteurs d'émission utilisable dans la présente invention,
- la figure 3 est une vue schématique en coupe illustrant un exemple de cavité mélangeuse pour un ou plusieurs transducteurs ultrasonores,
- la figure 4 est une vue en perspective de la partie interne de la cavité mélangeuse de la figure 3,
- la figure 5 est une vue schématique en perspective montrant le réseau de transducteurs d'émission et le réseau de transducteurs de réception assemblés sur un support,
- la figure 6 est un exemple de cartographie des vitesses de déplacement d'une surface mesurée par le dispositif des figures 1 à 5,
- la figure 7 est un exemple de cartographie des déplacements d'une surface mesurée par le dispositif des figures 1 à 5,
- la figure 8 est un exemple de courbe des vitesses de déplacement d'un point de la surface au cours du temps, mesurée par le dispositif des figures 1 à 5, et
- la figure 9 est un exemple de courbe des déplacements d'un point de la surface au cours du temps, mesurée par le dispositif des figures 1 à 5.

Sur les différentes figures, les mêmes références désignent des éléments identiques ou similaires.

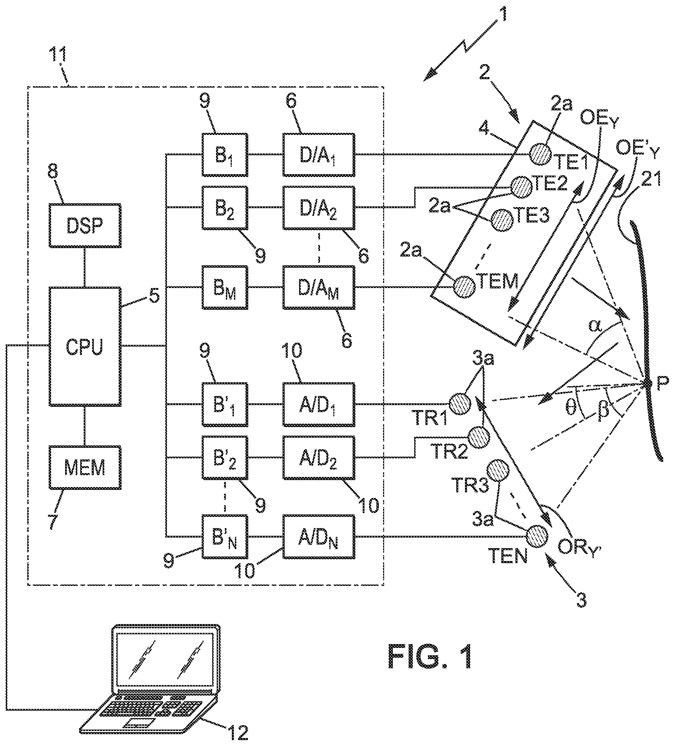

La figure 1 représente un dispositif ultrasonore 1 pour mesurer les mouvements d'une surface 21, qui peut être de toute nature, solide ou liquide. La surface 21 peut par exemple être la peau d'un être humain ou animal.

Le dispositif ultrasonore 1 comporte un réseau de transducteurs d'émission 2, qui peut comprendre un ou plusieurs transducteurs ultrasonores d'émission 2a au nombre de M (TE1, TE2, ... TEM), et un réseau de transducteurs de réception 3, qui comprend plusieurs transducteurs ultrasonores de réception 3a au nombre de N (TR1, TR2, ... TRN) .

Les deux réseaux de transducteurs 2, 3 peuvent avantageusement être des réseaux bidimensionnels.

Les deux réseaux 2, 3 peuvent éventuellement être disposés sur un même support et, auquel cas les transducteurs 2a, 3a peuvent être intercalés les uns entre les autres.

Les transducteurs 2a, 3a peuvent être de tout type connu. Dans un mode de réalisation, les transducteurs ultrasonores d'émission 2a peuvent être des haut-parleurs de bande passante élevée et les transducteurs ultrasonores de réception 3a peuvent être des microphones de bande passante élevée.

Le ou les transducteurs ultrasonores d'émission 2a peuvent éventuellement être disposés pour émettre des ondes ultrasonores dans une cavité mélangeuse 4 avant envoi vers la surface 21, comme il sera décrit plus loin.

Le réseau de transducteurs d'émission 2 et le réseau de transducteurs de réception 3 sont prévus pour mesurer les mouvements d'une pluralité de points de mesure P appartenant à la surface 21, par exemple pour mesurer les mouvements en sensiblement tout point de la surface 21 sur une aire supérieure à 10 cm$^2$ et pouvant être de plusieurs dizaines de cm$^2$.

A cet effet, Le réseau de transducteurs d'émission 2 et le réseau de transducteurs de réception 3 sont prévus pour avoir chacun une relativement grande ouverture, de façon que chaque point de mesure P de la surface 21 soit illuminé sous un grand nombre d'angles d'incidence par les ondes ultrasonores incidentes venant du réseau de transducteurs d'émission 2, et de façon que chaque point P de la surface 21 soit vu par les transducteurs de réception 3a sous un grand nombre d'angles de vue.

L'ouverture du réseau de transducteurs d'émission 2 peut être définie comme les dimensions transversales $OE_Y$, $OE_Z$ du réseau, par exemple selon un axe vertical Z et un axe horizontal transversal Y (voir les figures 1, 2, 5). Cette ouverture correspond à un angle d'ouverture en émission $\alpha$ sous lequel est vu le réseau de transducteurs d'émission 2 depuis chaque point de mesure P. Chaque point de mesure P est ainsi illuminé par les ondes ultrasonores incidentes sous une multiplicité d'angles d'incidence formant une plage d'angles d'incidence de largeur $\alpha$. L'angle d'ouverture $\alpha$ peut être par exemple au moins égal à 20 degrés.

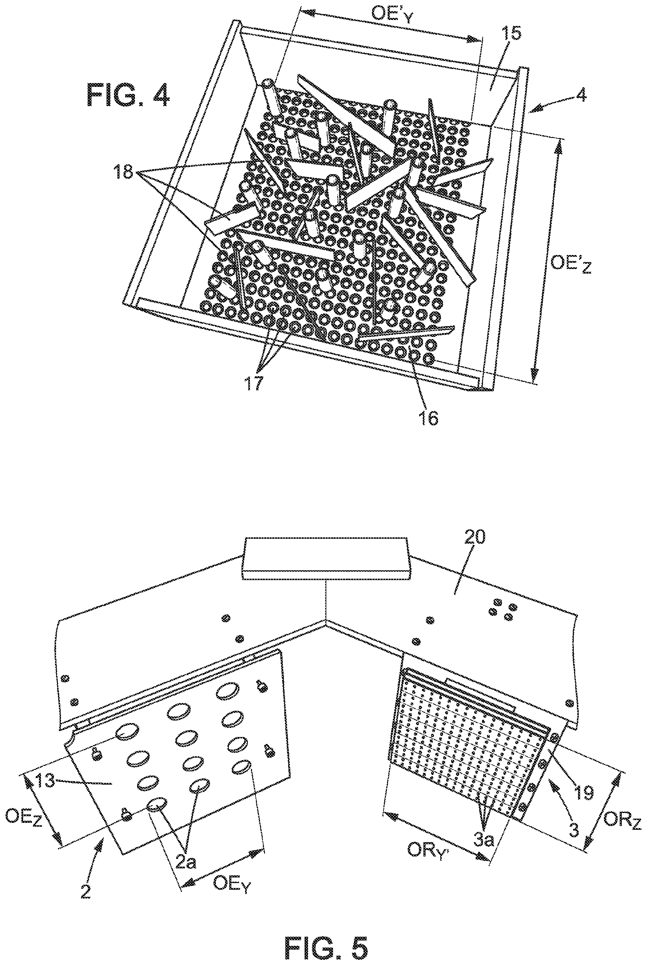

L'ouverture du réseau de transducteurs de réception 3 peut être définie comme les dimensions transversales $OR_{Y'}$, $OR_Z$ du réseau, par exemple selon un axe vertical Z et un axe horizontal transversal Y' (voir les figures 1, 5). Cette ouverture correspond à un angle d'ouverture en réception $\beta$ sous lequel est vu le réseau de transducteurs de réception 3 depuis chaque point de mesure P. Chaque point de mesure P est ainsi vu par les transducteurs ultrasonores de réception 3a sous une multiplicité d'angles de vue formant une plage d'angles de vue de largeur $\beta$. L'angle d'ouverture en réception $\beta$ peut être par exemple au moins égal à 20 degrés.

L'angle d'ouverture en émission $\alpha$ peut avantageusement être supérieur à l'angle d'ouverture en réception $\beta$.

Plus particulièrement, l'ouverture du réseau de transducteurs d'émission 2 selon au moins un axe Y, Z, ou les deux, est avantageusement supérieure à l'ouverture du réseau de transducteurs de réception selon les axes correspondant Y', Z. Cette disposition permet de s'assurer qu'au moins une partie des ondes ultrasonores réfléchies revient toujours vers le réseau de transducteurs de réception 3.

Eventuellement, l'ouverture du réseau de transducteurs d'émission 2 selon au moins un axe Y, Z, ou les deux, peut être supérieure à 3 fois (voire 5 fois, ou même 7 fois) l'ouverture du réseau de transducteurs de réception selon les axes correspondant Y', Z.

Le dispositif ultrasonore 1 peut être commandé par un circuit électronique 11 lui-même éventuellement relié à un ordinateur 12 ou similaire.

Le circuit électronique 11 peut comprendre :

-   un convertisseur digital-analogique 6 (D/A1-D/AM) relié à chaque transducteur d'émission 2a (E1-EM) et associé le cas échéant respectivement à une mémoire tampon 9 (B1-BN),
-   une unité centrale électronique 5 (CPU) commandée par l'ordinateur 12 et commandant chaque convertisseur analogique-digital 6,
-   au moins une mémoire centrale 7 (MEM) reliée à l'unité centrale électronique 5,
-   éventuellement, un circuit spécifique de traitement de signal 8 (DSP)
-   un convertisseur analogique-digital 10 (A/D1-A/DN) relié à chaque transducteur de réception 3a (TR1-TRN)), et associé respectivement à une mémoire tampon 9 (B'1-B'N).

Les transducteurs ultrasonores d'émission 2a peuvent être commandés pour émettre les ondes ultrasonores incidentes à une fréquence inférieure à 100 kHz, par exemple comprise entre 40 et 70 kHz.

Avantageusement, l'unité centrale électronique 5 est adaptée pour faire émettre les ondes ultrasonores incidentes à une cadence supérieure à 500 tirs par seconde, de façon à pouvoir suivre les mouvements de la surface 21 au cours du temps.

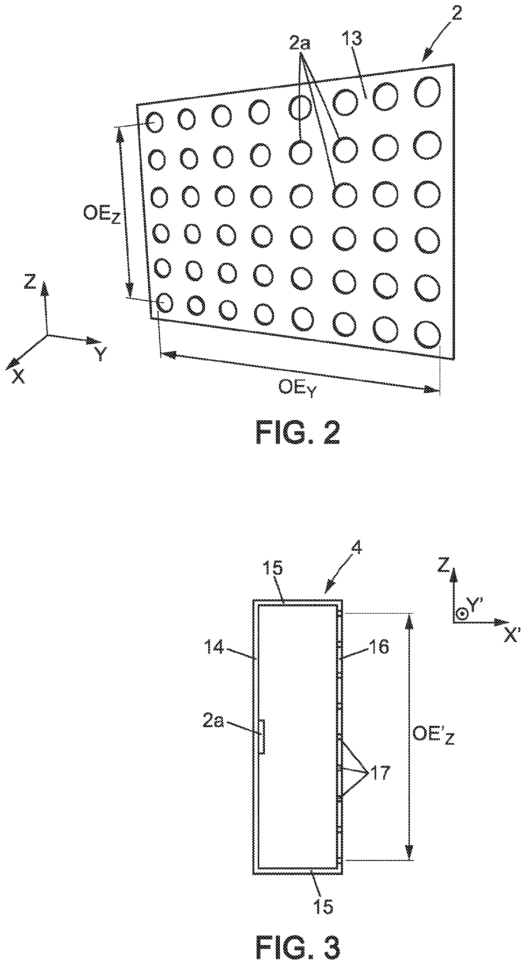

Comme représenté sur la figure 2 dans un exemple particulier de réalisation, le réseau de transducteurs d'émission 2 peut être un réseau bidimensionnel de transducteurs ultrasonores d'émission 2a portés par une plaque rigide 13.

Ces transducteurs ultrasonores d'émission 2a peuvent être commandés pour émettre les ondes ultrasonores incidentes soit en même temps, soit séquentiellement.

Eventuellement, les transducteurs ultrasonores d'émission 2a peuvent être répartis en plusieurs groupes et tous les transducteurs ultrasonores d'émission 2a d'un même groupe sont commandés pour émettre simultanément le même signal. On obtient ainsi un réseau démultiplié, ce qui augmente artificiellement l'ouverture en émission du réseau de transducteurs d'émission 2.

En variante ou en complément, le réseau de transducteurs d'émission 2 peut être couplé à une cavité mélangeuse 4, comme expliqué ci-dessus et comme représenté sur les figures 1 et 3. Le réseau de transducteurs d'émission 2 peut être éventuellement dans ce cas réduit à un seul transducteur ultrasonore d'émission 2a, puisque l'ouverture $OE'_Y$, $OE'_Z$ du réseau de transducteurs d'émission 2 est alors définie non par la répartition spatiale des transducteurs mais par les dimensions de la face émettrice de la cavité mélangeuse, par où les ondes ultrasonores incidentes sortent dans l'air vers la surface 21.

La cavité mélangeuse 4 peut être un objet solide tel qu'une plaque ou autre, ou bien il peut s'agir d'un boîtier dans lequel est monté le réseau de transducteurs d'émission 2, tel que représenté sur la figure 3. Dans ce cas, la cavité mélangeuse 4 peut comporter un fond 14, des parois latérales 15 et une paroi d'émission parallèle au fond 14 et maintenue écartée dudit fond 14 par les parois latérales 15. Le réseau de transducteurs d'émission 2 peut être monté sur le fond 14, dans la cavité mélangeuse 4. La paroi d'émission 16 peut comporter des trous 17 permettant la sortie des ondes ultrasonores incidentes vers la surface 21. Ces trous 17 constituent autant de sources d'ultrasons qui définissent l'ouverture du réseau de transducteurs d'émission 2.

Comme représenté sur la figure 4, à l'intérieur de la cavité mélangeuse peuvent être prévues des parois réfléchissantes 18, solidaires par exemple de la paroi d'émission 16 et avantageusement sensiblement perpendiculaires au fond 14. Les parois réfléchissantes 18 sont orientées de façons diverses pour favoriser les réflexions multiples des ondes ultrasonores dans la cavité mélangeuse 4.

Le dispositif qui vient d'être décrit permet de mesurer les mouvements de la surface 21, notamment le déplacement et la vitesse de déplacement en tout point.

Cette mesure est effectuée en traitant les signaux réfléchis captés par les transducteurs ultrasonores de réception 3a, par une méthode de traitement de signal qui peut par exemple être l'une des trois méthodes décrites ci-après.

**Méthode 1**

Dans cette première méthode de traitement de signal, l'illumination de la surface 21 par les ondes ultrasonores incidentes (le tir des ondes ultrasonores incidentes) est réalisée en excitant simultanément les M transducteurs ultrasonores d'émission 2a.

A chaque **étape de mesure** k, correspondant à un tir d'onde incidente, les signaux captés par les transducteurs ultrasonores de réception 3a sont traités par formation de voie en réception, de façon classique.

Ainsi, pour chaque point P de la zone d'observation (c'est-à-dire la zone susceptible d'être occupée par la surface 21), on calcule un signal de formation de voie en réception $S_k(t)$ comme suit :

$$S_k(t) = \sum_{j=1}^{N} r_j\left(t - \frac{d_j}{c}\right) \qquad (1)$$

Où :

- $r_j$ est le signal capté par le transducteur Rj du réseau de réception 3,
- t est le temps,
- $d_j$ est la distance entre le point P et le transducteur Rj,
- c est la célérité de l'onde ultrasonore dans l'air.

Ce signal est maximum pour les points P appartenant à la surface réfléchissante 21.

On peut ainsi déterminer une topographie de la surface 21, et donc une image de la surface 21, qui est cependant le plus souvent approximative.

En revanche, on peut déterminer de façon très précise les déplacements δ en chaque point P de la surface entre deux tirs k et k+1, par comparaison entre les signaux $S_k(t)$ correspondants aux différents tirs successifs.

Pour un point P donné de la surface 21, on compare les signaux successifs $S_k(t)$ et $S_{k+1}(t)$ lors d'au moins une **étape de détermination de mouvement,** ce qui fait apparaître un déphasage φ qui est relié au déplacement δ subi par le point P entre les tirs k et k+1 perpendiculairement à la surface 21, par la relation :

$$\frac{\varphi}{2\pi f} = \frac{2\delta cos\theta}{c} \qquad (1),$$

où :

- c est la célérité de l'onde ultrasonore dans l'air,
- f est la fréquence de l'onde ultrasonore
- θ est l'angle d'incidence et de réflexion moyen de l'onde ultrasonore au point P, c'est-à-dire l'angle moyen entre la normale à la surface et le faisceau réfléchi (déterminé en fonction de la topographie de la surface 21).

Compte tenu que la surface 21 est réfléchissante pour les ondes ultrasonores, le déplacement d de la surface 21 est vu comme un déplacement 2d.cosθ d'une source virtuelle située derrière la surface 21, d'où la formule (1) ci-dessus.

Le déplacement δ du point P entre les tirs k et k+1 est donc calculé par la formule :

$$\delta = \frac{c.\varphi}{4\pi f cos\theta} \qquad (2).$$

De façon équivalente, on peut mesurer non pas le déphasage $\varphi$, mais le retard $dt=\varphi/(2\pi f)$ du signal $S_{k+1}(t)$ par rapport à $S_k(t)$ (t est compté à chaque fois à partir du tir de l'onde onde incidente), le calcul du déplacement $\delta$ entre les tirs k et k+1 étant alors effectué par la formule :

$$\delta = \frac{c.dt}{2.cos\theta} \qquad (2').$$

Dans les deux cas, on peut aisément remonter à la vitesse normale de la surface 21 : $V = \delta /\Delta t$, où $\Delta t$ est l'intervalle de temps entre les tirs k et k+1.

Cette première méthode est très rapide et particulièrement efficace notamment si la surface 21 est illuminée sous une large plage angulaire et donc si le réseau de transducteurs d'émission 2 présente une ouverture d'émission large.

L'usage d'un réseau démultiplié ou d'une cavité mélangeuse est donc particulièrement souhaitable dans ce cas pour le réseau de transducteurs d'émission 2.

## Méthode 2

Dans cette deuxième méthode de traitement de signal, on n'utilise préférentiellement pas de cavité mélangeuse pour le réseau de transducteurs d'émission 2.

On fait émettre successivement une onde impulsionnelle successivement par chaque transducteur ultrasonore d'émission 2a, que l'on considérera comme ponctuel et émettant une onde sphérique. Chaque série de tirs successifs par l'ensemble des transducteurs ultrasonores d'émission 2a, constitue une **étape de mesure** k, puis on réitère les tirs dans une série suivante k+1 de tirs successifs par tous les transducteurs ultrasonores d'émission 2a.

A chaque série k de tirs, on détermine ainsi la réponse impulsionnelle $h_{ijk}(t)$ entre chaque transducteur ultrasonore d'émission Ei et chaque transducteur ultrasonore de réception TRj.

Pour un point P donné de la zone à observer, on connaît la distance à parcourir par l'onde ultrasonore de l'émetteur Ei au récepteur Rj, notée $d_{ij}$. Cette distance est connue pour tous les couples (Ei,Rj).

On calcule alors un signal de formation de voie (« beamforming ») en émission et réception au point P pour le transducteur ultrasonore de réception TRj. Ce signal $r_{jk}(t)$ est obtenu en sommant toutes les réponses impulsionnelles retardées de manière à compenser les temps de trajet dij /c. Ce signal s'exprime comme suit :

$$r_{jk}(t) = \sum_{i=1}^{M} h_{ijk}\left(t - \frac{d_{ij}}{c}\right) \qquad (3)$$

Le signal $S'_k(t)$ correspondant au point P et à la série k de tirs, pour l'ensemble des transducteurs ultrasonores de réception 3a, est la sommation des signaux de formation de voie de tous les récepteurs :

$$S'_k(t) = \sum_{j=1}^{N} r_{jk}(t) \qquad (4)$$

Si P est un point matériel de la surface 21, alors le signal S sera une impulsion brève et d'amplitude maximum car toutes les impulsions calculées pour tous les récepteurs auront le même retard. Sinon le signal sera plus étalé en temps et d'amplitude plus faible.

Ce procédé permet dans une première approche de déterminer la topographie de la surface 21, donc une image (généralement peu précise) de la surface 21.

Pour un même point P de la surface, les signaux successifs $S'_k(t)$ correspondant aux différentes étapes de mesure k permettent de déterminer, avec une grande précision, le déplacement $\delta$ du point P entre deux séries successives de tirs k et k+1, au cours d'au moins une **étape de détermination de mouvement.**

Dans toutes les formes de réalisation de l'invention, les étapes de détermination de mouvement peuvent être réalisées au fur et à mesure des étapes de mesure. Bien entendu, ce calcul peut aussi être fait de façon différée.

Si le point P a bougé entre t et t+dt d'une distance $\delta$ normale à la surface 21, alors le nouveau signal $S'_{k+1}(t)$ sera une impulsion décalée en temps (compté par rapport au tir des ondes incidentes) par rapport à $S'_k(t)$ de $dt=2\delta \cos\theta$, avec les notations déjà définies ci-dessus.

Le décalage temporel dt donne le déplacement de la surface 21 au point P :

$$\delta = dt.c \ / (2 \ \cos\theta) \qquad (5).$$

On peut aisément remonter à la vitesse normale de la surface : $V = \delta /\Delta t$, où $\Delta t$ est l'intervalle de temps entre les séries de tirs k et k+1.

Comme dans la méthode 1, on peut déterminer de façon équivalente le déplacement et la vitesse de la surface 21 en utilisant le déphasage $\varphi$ entre les signaux $S'_k(t)$ et $S'_{k+1}(t)$.

La méthode 2 est très précise et peut permettre des mesures de mouvement de surface d'amplitude largement inférieure à la longueur d'onde. Une interpolation parabolique ou d'ordre supérieur pourra alors être mise en œuvre pour calculer le décalage temporel à la fraction près de la période d'échantillonnage des signaux acoustiques.

**Méthode 3**

Dans cette troisième méthode, le réseau de transducteurs d'émission 2 peut être de type ouverture démultipliée ou comporter une cavité mélangeuse 4.

Les ondes émises réfléchissent sur la surface 21 et sont collectées par les N transducteurs ultrasonores de réception 3a. On considérera le trajet des ondes de la surface 21 vers les récepteurs comme étant direct sans aucune réflexion.

Cette méthode 3 comporte au moins une **étape de calibrage** (préliminaire, et pouvant être répétée à intervalle régulier pour recalibrer) où on détermine les réponses impulsionnelles $h_{ij}(t)$ entre les transducteurs ultrasonores d'émission TEi et les transducteurs ultrasonores de réception TRj. Après une transformée de Fourier, ces réponses impulsionnelles sont notées $H_{ij}$ (on omet la pulsation angulaire $\omega$ pour simplifier les calculs). Cette étape préliminaire peut éventuellement être réalisée en faisant émettre une impulsion successivement par chaque transducteur ultrasonore d'émission TEi, comme dans la méthode 2 ci-dessus.

Cette étape préliminaire est suivie de plusieurs **étapes de mesure** successives, indexées par un indexe k. Pendant chaque étape de mesure k, on fait focaliser l'onde incidente successivement sur les différents points P de la surface 21, lesquels points P ont par exemple été déterminés au cours de l'étape préliminaire.

Ces focalisations successives sont réalisées comme suit.

Suivant le procédé de retournement temporel et en usant du principe de réciprocité, en faisant émettre par l'émetteur i la réponse impulsionnelle temporellement inversée (donc dans le domaine fréquentiel, $H_{ij}^*$, où l'exposant * désigne le complexe conjugué) on devrait obtenir la réponse suivante : $R_{ij} = H_{ij} H_{ij}^* = |H_{ij}|^2$. La réponse $R_{ij}$ est réelle et correspond à une impulsion centrée sur l'origine des temps. Suivant ce constat il est donc possible d'envoyer une impulsion à n'importe lesquels des récepteurs j avec un certain retard en émettant simplement la réponse impulsionnelle retournée temporellement et retardée.

De fait, il est possible d'émettre n'importe quel front d'onde vers tous les récepteurs à partir d'un seul émetteur i. Ce front d'onde étant décrit simplement par un retard d'arrivée noté $T_j$ au récepteur j. Pour cela il suffit de combiner les signaux avant de les faire réémettre par le transducteur ultrasonore d'émission TEi :

$$E_i = \sum_{j=1}^{N} H_{ij}^* e^{-j\omega T_j} \quad (6),$$

où $E_i$ est le signal émis par le transducteur ultrasonore d'émission TEi, dans le domaine fréquentiel.

On peut donc notamment synthétiser un front d'onde sphérique arrivant au niveau des transducteurs ultrasonores de réception TRj, issu d'un centre de divergence constitué par un point P sur la surface 21. Autrement dit, on peut focaliser l'onde ultrasonore incidente au point P sur la surface 21.

Cette synthèse de front d'onde est par ailleurs renforcée lorsque tous les transducteurs ultrasonores d'émission TEi émettent les signaux nécessaires à la synthèse du même front d'onde. En notation matricielle les signaux émis par tous les transducteurs ultrasonores d'émission TEi peuvent s'écrire sous la forme suivante :

$$E = \begin{bmatrix} E_1 \\ E_2 \\ \vdots \\ E_M \end{bmatrix} = \begin{pmatrix} H_{11}^* & \cdots & H_{1N}^* \\ \vdots & \ddots & \vdots \\ H_{M1}^* & \cdots & H_{MN}^* \end{pmatrix} \begin{bmatrix} e^{-j\omega T_1} \\ e^{-j\omega T_2} \\ \vdots \\ e^{-j\omega T_N} \end{bmatrix} \quad (7)$$

Au niveau des transducteurs ultrasonores de réception TRj, les signaux reçus sont :

$$R_j = \sum_{i=1}^{M} E_i H_{ij} \quad (8)$$

Sous forme matricielle cela se résume à :

$$R = \begin{bmatrix} R_1 \\ R_2 \\ \vdots \\ R_M \end{bmatrix} = \begin{pmatrix} H_{11} & \cdots & H_{M1} \\ \vdots & \ddots & \vdots \\ H_{1N} & \cdots & H_{MN} \end{pmatrix} \begin{bmatrix} E_1 \\ E_2 \\ \vdots \\ E_M \end{bmatrix} = HE \quad (9)$$

On peut combiner tous les signaux reçus par les différents transducteurs ultrasonores de réception TRj après focalisation au point P de la surface 21, en réalisant une formation de voie en réception (beamforming) grâce à la compensation des retards des signaux reçus avant leur sommation :

$$S_k = \sum_{j=1}^{N} R_j e^{+j\omega T_j} = [e^{+j\omega T_1} \quad e^{+j\omega T_2} \quad \ldots \quad e^{+j\omega T_N}]R = TR \quad (10)$$

L'indice k désigne l'étape de mesure, c'est-à-dire une succession de tirs d'ondes incidentes successivement focalisées sur les différents points P considérés de la surface 21.

En notation matricielle, la focalisation au point P en émission et la formation de voie en réception se résume à l'unique signal suivant :

$$S_k = T\ R = T\ H\ E = T\ H\ H^{T^*}T^{T^*} \quad (11)$$

L'exposant T* indique que la matrice est transposée conjuguée.

Le signal $S_k$ représente principalement les informations collectées au point P.

Lorsque ce point bouge entre l'étape de mesure k et l'étape de mesure k+1, il en résulte un déphasage $\varphi$ entre $S_k$ et $S_{k+1}$.

Ce déphasage $\varphi$ permet de remonter au déplacement $\delta$ de la surface 21 au point P par la formule (2) susmentionnée, ou à la vitesse de déplacement V comme expliqué dans la méthode 1, au cours d'au moins une **étape de détermination de mouvement.**

On notera que les calculs ci-dessus de la troisième méthode pourraient être faits de même dans le domaine temporel plutôt que dans le domaine fréquentiel. Inversement, les calculs des méthodes 1 et 2 pourraient également éventuellement être faits dans le domaine fréquentiel.

Dans les différentes méthodes envisagées, selon les applications, les valeurs de $\delta$ et V pourraient être des valeurs proportionnelles aux formules susmentionnées.

**Variante**

La formation de voie en réception peut être éventuellement réalisée avec un mélangeur, comme explicité dans les documents suivants :

- Nicolas Quieffin, Stefan Catheline, Ros Kiri Ing and Mathias Fink, "Acoustic source localization model using in-skull reverberation and time reversal", Applied Physics Letters vol. 90, 063902 (2007) ;

N. Etaix, M. Fink and R. K. Ing, "Acoustic imaging device with one transducer", J. Acoust. Soc. Am. 131 (5), pp. EL395-EL399, 2012 ;
N. Etaix, J. Dubois, M. Fink and R. K. Ing, "Increasing the modal density in plates for mono-element focusing in air", J. Acoust. Soc. Am., Vol. 134 (2), pp. 1049-1054, 2013.

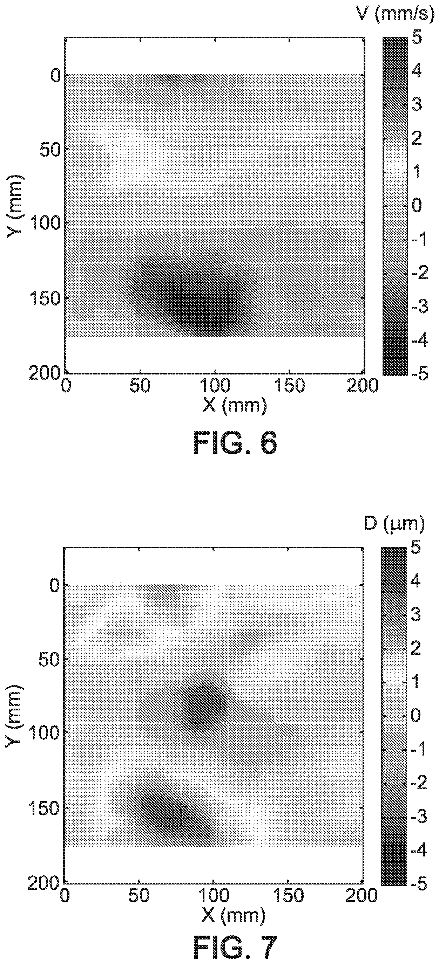

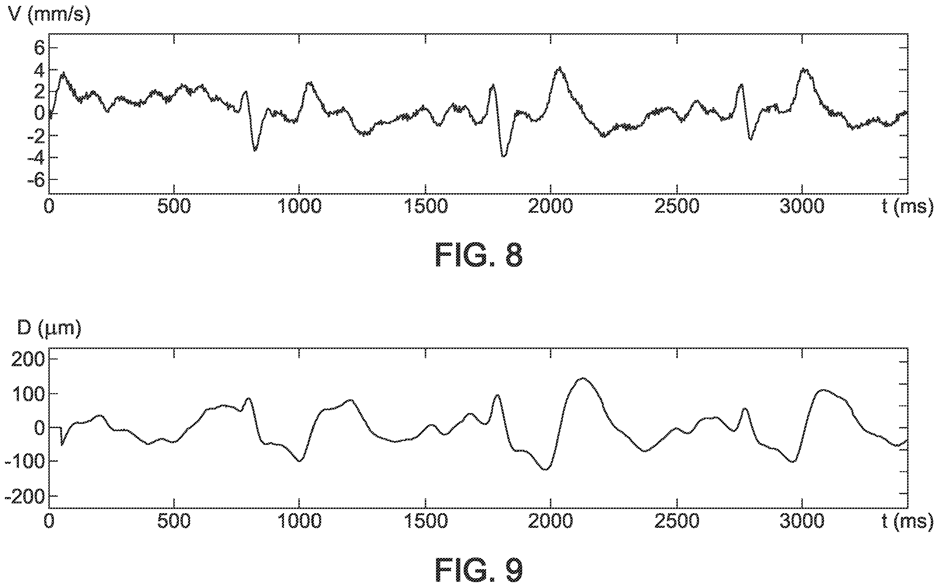

Les figures 6 et 7 montrent un exemple de cartographie des vitesses de déplacement et des déplacements mesurés par le procédé selon l'invention sur la surface 21 du bas du sternum du corps humain, mise en vibration par les battements cardiaques. Les figures 8 et 9 montrent l'évolution dans le temps des vitesses de déplacement et des déplacements mesurés par le procédé selon l'invention sur la même surface solide 21.

L'invention est donc particulièrement utile pour l'analyse des mouvements de surface du corps humain, notamment pour l'étude des mouvements respiratoires ou du système cardiovasculaire.

L'invention est particulièrement avantageuse notamment dans l'étude de la ventilation pulmonaire, puisqu'elle permet de remonter aux variations de volume des poumons et donc aux débits d'air inspiré / expiré sans perturber le sujet, contrairement aux procédés impliquant par exemple un embout buccal ou un masque facial.

L'invention permet également de surveiller de façon automatique la respiration d'un patient, par exemple en réanimation, en réveil d'anesthésie, en salle d'attente aux urgences, ou encore pour les nourrissons à risque, etc.

En dehors du domaine médical, l'invention peut également permettre par exemple de détecter précocement un endormissement d'une personne, de détecter une situation de stress d'une personne, etc.

Enfin en dehors du domaine médical, l'invention peut également permettre de détecter des objets fixés sur la peau et cachés par un tissus. En excitant le sujet et plus particulièrement la zone observée le mouvement de surface de la peau sera perturbé par le ou les objets cachés et sera observable par le dispositif revendiqué dans ce document.

**Revendications**

1. Procédé pour détecter des mouvements d'une surface (21) réfléchissant les ondes ultrasonores, comprenant plusieurs étapes de mesure successives au cours de chacune desquelles on émet au moins une onde ultrasonore incidente dans l'air vers la surface (21) avec un dispositif d'émission (2) d'ondes ultrasonores et on capte des signaux réfléchis représentatifs d'au moins une onde ultrasonore réfléchie dans l'air par ladite surface (21) à partir de ladite au moins une onde ultrasonore incidente, **caractérisé en ce qu'**au cours de chaque étape de mesure :

   - on mesure les mouvements d'une pluralité de points de mesure (P) appartenant au moins à ladite surface en illuminant chaque point de mesure (P) par ladite au moins une onde ultrasonore incidente sous une multiplicité d'angles d'incidence,
   - on capte les signaux réfléchis avec un réseau de transducteurs de réception (3) comprenant une pluralité de transducteurs ultrasonores de réception (3a) et on détermine un signal de formation de voie pour chaque point de mesure (P), par formation de voie au moins en réception à partir desdits signaux réfléchis,

   **et en ce qu'**il comporte en outre au moins une étape de détermination de mouvement au cours de laquelle on détermine lesdits mouvements de la surface (21) au point de mesure (P) considéré en déterminant au moins un retard ou un déphasage entre deux signaux de formation de voie pour ce point de mesure (P).

2. Procédé selon la revendication 1, dans lequel au cours de chaque étape de mesure, on illumine chaque point de mesure (P) de la surface (21) avec ladite au moins une onde incidente sous des angles d'incidence s'étendant sur une plage d'angles d'incidence d'au moins à 20 degrés.

3. Procédé selon la revendication 1 ou la revendication 2, dans lequel au cours de chaque étape de mesure, on mesure les mouvements en sensiblement tout point de la surface (21) sur une aire supérieure à 10 cm$^2$.

4. Procédé selon l'une quelconque des revendications précédentes, dans lequel le dispositif d'émission (2) d'ondes ultrasonores et le réseau de transducteurs de réception (3) sont bidimensionnels.

**5.** Procédé selon la revendication 4, dans lequel le réseau de transducteurs d'émission (2) présente une ouverture (OE$_Y$, OE$_Z$, OE'$_Y$, OE'$_Z$) au moins égale à une ouverture (OR$_{Y'}$, OR$_Z$) du dispositif de réception (3) d'ondes ultrasonores selon deux directions sensiblement perpendiculaires.

**6.** Procédé selon la revendication 5, dans lequel l'ouverture (OE$_Y$, OE$_Z$, OE'$_Y$, OE'$_Z$) du réseau de transducteurs d'émission (2) est au moins égale à trois fois l'ouverture (OR$_{Y'}$, OR$_Z$) du dispositif de réception (3) d'ondes ultrasonores, selon au moins une des directions.

**7.** Procédé selon l'une quelconque des revendications 4 à 6, dans lequel l'ouverture (OE$_Y$, OE$_Z$, OE'$_Y$, OE'$_Z$) du dispositif d'émission (2) d'ondes ultrasonores est au moins égale à 20 cm dans chaque direction.

**8.** Procédé selon l'une quelconque des revendications précédentes, dans lequel le dispositif d'émission (2) d'ondes ultrasonores comprend un réseau de transducteurs d'émission (2) comprenant une pluralité de transducteurs ultrasonores d'émission (2a).

**9.** Procédé selon la revendication 8, dans lequel les transducteurs ultrasonores d'émission (2a) sont répartis en plusieurs groupes et au cours de ladite étape de mesure, on fait émettre simultanément un même signal par les transducteurs ultrasonores d'émission (2a) appartenant à un même groupe.

**10.** Procédé selon l'une quelconque des revendications précédentes, dans lequel le dispositif d'émission (2) d'ondes ultrasonores comprend au moins un transducteur ultrasonore d'émission (2a) disposé pour émettre dans une cavité mélangeuse (4) configurée pour provoquer des réflexions multiples de ladite au moins une onde ultrasonore incidente avant envoi vers la surface (21) .

**11.** Procédé selon l'une quelconque des revendications précédentes, dans lequel les ondes ultrasonores ont une fréquence inférieure à 100 kHz.

**12.** Procédé selon l'une quelconque des revendications précédentes, dans lequel les ondes ultrasonores sont émises à une cadence supérieure à 500 tirs par seconde.

**13.** Procédé selon l'une quelconque des revendications précédentes, dans lequel au cours de chaque étape de mesure d'indice k, on calcule au moins en différents point (P) de la surface (21) un signal de formation de voie S$_k$ en réception audit point (P),
et au cours de chaque étape de détermination de mouvement, on détermine le mouvement de chaque point (P) de la surface (21) en déterminant un retard ou un déphasage entre les signaux formation de voie S$_k$ en réception audit point (P), pour deux valeurs de k différentes.

**14.** Procédé selon la revendication 13, dans lequel au cours de chaque étape de mesure d'indice k, le dispositif d'émission (2) d'ondes ultrasonores émet une onde ultrasonore incidente non focalisée vers la surface (21) .

**15.** Procédé selon la revendication 13, dans lequel au cours de chaque étape de mesure d'indice k, le dispositif d'émission (2) d'ondes ultrasonores émet successivement des ondes ultrasonore incidente focalisées vers les différents points (P) de la surface (21) et le signal de formation de voie S$_k$ en réception audit point (P) est déterminé à partir des signaux réfléchis correspondant à l'onde ultrasonore incidente focalisée audit point (P).

**16.** Procédé selon l'une quelconque des revendications 13 à 15, dans lequel le signal de formation de voie en réception S$_k$ est déterminé par la formule :

$$S_k(t) = \sum_{j=1}^{N} r_j \left( t - \frac{d_j}{C} \right)$$

où :

- r$_j$ est le signal capté par le transducteur ultrasonore de réception (3a) d'indice j,
- t est le temps,
- d$_j$ est une distance entre le point P et le transducteur ultrasonore de réception (3a) d'indice j,

- c est la célérité de l'onde ultrasonore dans l'air.

**17.** Procédé selon l'une quelconque des revendications 1 à 12, dans lequel le dispositif d'émission (2) d'ondes ultrasonores comprend un réseau de transducteurs d'émission (2) comprenant une pluralité de transducteurs ultrasonores d'émission (2a), et dans lequel

au cours de chaque étape de mesure d'indice k, on mesure des réponses impulsionnelles respectives entre chaque transducteur ultrasonore d'émission (2a) et chaque transducteur ultrasonore de réception (3a), puis on calcule au moins en différents point (P) de la surface (21) un signal de formation de voie $S'_k$ en émission et réception audit point (P),

et au cours de chaque étape de détermination de mouvement, on détermine un mouvement de chaque point (P) de la surface (21) en déterminant un retard ou un déphasage entre les signaux de voie $S'_k$ en émission et réception audit point (P), pour deux valeurs de k différentes.

**18.** Procédé selon la revendication 17, dans lequel chaque signal de formation de voie en émission et réception audit point (P) est déterminé par la formule :

$$S'_k(t) = \sum_{j=1}^{N} \sum_{i=1}^{M} h_{ijk}\left(t - \frac{d_{ij}}{c}\right) \; ,$$

où :

- i est un indice compris entre 1 et M désignant un transducteur ultrasonore d'émission (2a),
- j est un indice compris entre 1 et N désignant un transducteur ultrasonore de réception (3a),
- $h_{ijk}$ est la réponse impulsionnelle entre le transducteur ultrasonore d'émission (2a) d'indice i et le transducteur ultrasonore de réception (3a) d'indice j,
- t est le temps,
- $d_{ij}$ est une distance parcourue par une onde ultrasonore depuis le transducteur ultrasonore d'émission (2a) d'indice i jusqu'au transducteur ultrasonore de réception (3a) d'indice j en se réfléchissant au point (P) considéré de la surface (21),
- c est la célérité des ondes ultrasonores dans l'air.

**19.** Procédé selon l'une quelconque des revendications précédentes, dans lequel au cours d'au moins certaines étapes de mesure, on détermine un signal de formation de voie pour chaque point de mesure (P) d'une zone d'observation prédéterminée et on détermine les points de mesure (P) appartenant à la surface (21) comme étant ceux qui maximisent le signal de formation de voie.

**20.** Procédé selon l'une quelconque des revendications précédentes, dans lequel au cours de l'étape de détermination de mouvement, on détermine un retard dt entre des signaux de formation de voie correspondant à deux étapes de mesures au même point de mesure (P) et on détermine :

- un déplacement δ au point de mesure (P) comme étant proportionnel dt.c
- et / ou une vitesse au point de mesure (P) comme étant proportionnelle à dt.c/Δt,

où c est la célérité des ondes ultrasonores dans l'air et Δt est un intervalle de temps entre lesdites deux étapes de mesure.

**21.** Procédé selon l'une quelconque des revendications 1 à 19, dans lequel au cours de l'étape de détermination de mouvement, on détermine un déphasage φ entre des signaux de formation de voie correspondant à deux étapes de mesures au même point de mesure (P) et on détermine :

- un déplacement δ au point de mesure (P) comme étant proportionnel c.φ/(2.π.f)
- et / ou une vitesse au point de mesure (P) comme étant proportionnelle à c.φ/(2.π.f)/Δt,

où c est la célérité des ondes ultrasonores dans l'air, f la fréquence des ondes ultrasonores et Δt est un intervalle de temps entre lesdites deux étapes de mesure.

**22.** Dispositif pour détecter des mouvements d'une surface (21) réfléchissant les ondes ultrasonores, comprenant :

un dispositif d'émission (2) d'ondes ultrasonores,
un dispositif de réception (3) d'ondes ultrasonores,
un dispositif de commande (5) commandant le dispositif d'émission (2) d'ondes ultrasonores et recevant des signaux captés par le dispositif de réception (3) d'ondes ultrasonores, dans lequel

le dispositif de commande (5) est configuré pour réaliser plusieurs étapes de mesure successives au cours de chacune desquelles le dispositif d'émission (2) d'ondes ultrasonores émet au moins une onde ultrasonore incidente dans l'air vers la surface (21) et le dispositif de réception (3) d'ondes ultrasonores capte des signaux réfléchis représentatifs d'au moins une onde ultrasonore réfléchie dans l'air par ladite surface (21) à partir de ladite au moins une onde ultrasonore incidente,

le dispositif de réception (3) d'ondes ultrasonores est un réseau de transducteurs de réception (3) comprenant une pluralité de transducteurs ultrasonores de réception (3a),

**le dispositif étant caractérisé en ce que le** dispositif d'émission (2) d'ondes ultrasonores est configuré pour illuminer une pluralité de point de mesure (P) appartenant au moins à ladite surface (21) par ladite au moins une onde ultrasonore incidente sous une multiplicité d'angles d'incidence,

**en ce que** le dispositif de commande (5) est configuré pour, au cours de chaque étape de mesure, déterminer un signal de formation de voie pour chaque point de mesure (P), par formation de voie au moins en réception à partir desdits signaux réfléchis,

**et en ce que** le dispositif de commande (5) est configuré pour déterminer lesdits mouvements de la surface (21) au point de mesure (P) considéré en déterminant au moins un retard ou un déphasage entre deux signaux de formation de voie pour ce point de mesure (P).

## Patentansprüche

1. Verfahren zum Erkennen von Bewegungen einer Oberfläche (21), die Ultraschallwellen reflektieren, mit mehreren aufeinanderfolgenden Messschritten, bei denen jeweils mindestens eine einfallende Ultraschallwelle mit einer Ultraschallwellenemissionsvorrichtung (2) in Richtung der Oberfläche (21) in die Luft emittiert wird und reflektierte Signale erfasst werden, welche ausgehend von der mindestens einen einfallenden Ultraschallwelle mindestens eine von der Oberfläche (21) in die Luft reflektierte Ultraschallwelle repräsentieren, **dadurch gekennzeichnet, dass** in jedem Messschritt:

   - die Bewegungen mehrerer mindestens zur Oberfläche (21) gehörenden Messpunkte (P) gemessen werden, indem jeder Messpunkt (P) mit der mindestens einen einfallenden Ultraschallwelle unter mehreren Einfallswinkeln bestrahlt wird,
   - die reflektierten Signale mit einem Netz von Empfangswandlern (3) erfasst werden, das mehrere Ultraschallempfangswandler (3a) aufweist, und ein Kanalbildungssignal für jeden Messpunkt (P) durch Bilden zumindest von Empfangskanälen anhand der reflektierten Signale ermittelt wird,

   und dass es ferner mindestens einen Schritt der Bewegungsermittlung aufweist, bei welchem die Bewegungen der Oberfläche (21) am betrachteten Messpunkt (P) ermittelt werden, indem mindestens eine Verzögerung oder eine Phasenverschiebung zwischen zwei Kanalbildungssignalen für diesen Messpunkt (P) ermittelt wird.

2. Verfahren nach Anspruch 1, bei welchem in jedem Messschritt jeder Messpunkt (P) der Oberfläche (21) mit der mindestens einen einfallenden Welle unter Einfallswinkeln bestrahlt wird, die sich über einen Einfallswinkelbereich von mindestens 20 Grad erstrecken.

3. Verfahren nach Anspruch 1 oder Anspruch 2, bei welchem in jedem Messschritt die Bewegungen an im Wesentlichen jedem Punkt der Oberfläche (21) in einer Fläche von mehr als 10 cm$^2$ gemessen werden.

4. Verfahren nach einem der vorhergehenden Ansprüche, bei welchem die Ultraschallwellenemissionsvorrichtung (2) und das Empfangswandlernetz (3) zweidimensional sind.

5. Verfahren nach Anspruch 4, bei welchem das Emissionswandlernetz (2) in zwei im Wesentlichen senkrechten Richtungen eine Apertur ($OE_Y$, $OE_Z$, $OE'_Y$, $OE'_Z$) aufweist, die mindestens gleich einer Apertur ($OR_Y$, $OR_Z$) der Ultraschallwellenempfangsvorrichtung (3) sind.

6. Verfahren nach Anspruch 5, bei welchem die Apertur ($OE_Y$, $OE_Z$, $OE'_Y$, $OE'_Z$) des Emissionswandlernetzes (2) in mindestens einer der Richtungen mindestens gleich dem Dreifachen der Apertur ($OR_Y$, $OR_Z$) der Ultraschallwellenempfangsvorrichtung (3).

7. Verfahren nach einem der Ansprüche 4 bis 6, bei welchem die Apertur ($OE_Y$, $OE_Z$, $OE'_Y$, $OE'_Z$) des Emissionswandlernetzes (2) in jeder Richtung mindestens gleich 20 cm ist.

8. Verfahren nach einem der vorhergehenden Ansprüche, bei welchem die Ultraschallwellenemissionsvorrichtung (2) ein Emissionswandlernetz (2) mit mehreren Ultraschallemissionswandlern (2a) aufweist.

9. Verfahren nach Anspruch 8, bei welchem die Ultraschallemissionswandler (2a) in mehreren Gruppen verteilt sind, und bei welchem während des Messschritts die zu derselben Gruppe gehörenden Ultraschallemissionswandler (2a) gleichzeitig dasselbe Signal ausgeben.

10. Verfahren nach einem der vorhergehenden Ansprüche, bei welchem die Ultraschallwellenemissionsvorrichtung (2) mindestens einen Ultraschallemissionswandler (2a) aufweist, der zum Emittieren in einem Mischhohlraum (4) angeordnet ist, welcher dazu ausgebildet ist, mehrere Reflexionen der mindestens einen einfallenden Ultraschallwelle vor dem Senden zu der Oberfläche (21) zu bewirken.

11. Verfahren nach einem der vorhergehenden Ansprüche, bei welchem die Ultraschallwellen eine Frequenz unter 100 kHz aufweisen.

12. Verfahren nach einem der vorhergehenden Ansprüche, bei welchem die Ultraschallwellen mit einer Kadenz von mehr als 500 Auslösungen pro Sekunde emittiert werden.

13. Verfahren nach einem der vorhergehenden Ansprüche, bei welchem in jedem Schritt des Messens des Index k zumindest an unterschiedlichen Punkten (P) der Oberfläche (21) ein Empfangskanalbildungssignal $S_k$ an diesem Punkt (P) berechnet wird, und in jedem Bewegungsermittlungsschritt die Bewegung jedes Punktes (P) der Oberfläche (21) ermittelt wird, indem eine Verzögerung oder Phasenverschiebung zwischen den Empfangskanalbildungssignalen $S_k$ an dem Punkt (P) für zwei unterschiedliche Werte von k ermittelt wird.

14. Verfahren nach Anspruch 13, bei welchem in jedem Schritt des Messens des Index k die Ultraschallwellenemissionsvorrichtung (2) eine nicht fokussierte einfallende Ultraschallwelle in Richtung der Oberfläche (21) emittiert.

15. Verfahren nach Anspruch 13, bei welchem in jedem Schritt des Messens des Index k die Ultraschallwellenemissionsvorrichtung (2) nacheinander fokussierte einfallende Ultraschallwellen in Richtung der verschiedenen Punkte (P) der Oberfläche (21) emittiert, und bei welchem das Empfangskanalbildungssignal $S_k$ an dem Punkt (P) anhand der reflektierten Signale entsprechend der fokussierten einfallenden Ultraschallwelle an dem Punkt (P) ermittelt wird.

16. Verfahren nach einem der Ansprüche 13 bis 15, bei welchem das Empfangskanalbildungssignal $S_k$ anhand der Formel

$$S_k(t) = \sum_{j=1}^{N} r_j \left( t - \frac{d_j}{c} \right)$$

ermittelt wird, wobei:

- $r_j$ das von dem Ultraschallempfangswandler (3a) mit dem Index j erfasste Signal bezeichnet,
- t die Zeit bezeichnet,
- $d_j$ eine Entfernung zwischen dem Punkt P und dem Ultraschallempfangswandler (3a) mit dem Index j bezeichnet,
- c die Geschwindigkeit der Ultraschallwelle in der Luft bezeichnet.

17. Verfahren nach einem der Ansprüche 1 bis 12, bei welchem die Ultraschallwellenemissionsvorrichtung (2) ein Emissionswandlernetz (2) mit mehreren Ultraschallemissionswandlern (2a) aufweist, und bei welchem in jedem Schritt des Messens des Index k jeweilige Impulsantworten zwischen jedem Ultraschallemissionswandler (2a) und jedem Ultraschallempfangswandler (3a) gemessen wird, wonach zumindest an verschiedenen Punkten (P) der Oberfläche (21) ein Emissions- und Empfangskanalbildungssignal S'$_k$ an dem Punkt (P) berechnet wird, und in jedem Schritt der Bewegungsermittlung eine Bewegung jedes Punktes (P) der Oberfläche (21) ermittelt wird, indem eine Verzögerung oder eine Phasenverschiebung zwischen den Emissions- und Empfangskanalsignalen S'$_k$ an dem Punkt (P) für zwei verschiedene Werte von k ermittelt wird.

18. Verfahren nach Anspruch 17, bei welchem jedes ein Emissions- und Empfangskanalbildungssignal an dem Punkt (P) anhand der Formel

$$S'_k(t) = \sum_{j=1}^{N} \sum_{i=1}^{M} h_{ijk}\left(t - \frac{d_{ij}}{c}\right)$$

ermittelt wird, wobei:

- i ein Index zwischen 1 und M ist, der einen Ultraschallemissionswandler (2a) bezeichnet,
- j ein Index zwischen 1 und N ist, der einen Ultraschallempfangswandler (3a) bezeichnet,
- h$_{ijk}$ die Impulsantwort zwischen dem Ultraschallemissionswandler (2a) mit dem Index i und dem Ultraschallempfangswandler (3a) mit dem Index j bezeichnet,
- t die Zeit bezeichnet,
- d$_j$ eine von einer Ultraschallwelle von dem Ultraschallemissionswandler (2a) mit dem Index i zu dem Ultraschallempfangswandler (3a) mit dem Index j unter Reflektion an dem betreffenden Punkt (P) der Oberfläche (21) zurückgelegte Entfernung angibt,
- c die Geschwindigkeit der Ultraschallwellen in der Luft bezeichnet.

19. Verfahren nach einem der vorhergehenden Ansprüche, bei welchem in zumindest einigen Messschritten ein Kanalbildungssignal für jeden Messpunkt (P) einer vorermittelten Beobachtungszone ermittelt wird und die zu der Oberfläche (21) gehörenden Messpunkte (P) als diejenigen ermittelt werden, welche das Kanalbildungssignal maximieren.

20. Verfahren nach einem der vorhergehenden Ansprüche, bei welchem die dem Schritt der Bewegungsermittlung eine Verzögerung dt zwischen den Kanalbildungssignalen, die zwei Messschritten an demselben Messpunkt (P) entsprechen, ermittelt wird und

- eine Verschiebung δ an dem Messpunkt (P) als proportional dt.c ermittelt wird
- und/oder eine Geschwindigkeit an dem Messpunkt (P) als proportional zu dt.c/Δt ermittelt wird,

wobei c die Geschwindigkeit der Ultraschallwellen in der Luft angibt und Δt ein Zeitintervall zwischen den beiden Messschritten ist.

21. Verfahren nach einem der Ansprüche 1 bis 19, bei welchem in dem Schritt der Bewegungsermittlung eine Phasen-verschiebung φ zwischen den Kanalbildungssignalen, die zwei Messschritten an demselben Messpunkt (P) entsprechen, ermittelt wird und

- eine Verschiebung δ an dem Messpunkt (P) als proportional c.(φ/(2.π.f) ermittelt wird
- und/oder eine Geschwindigkeit an dem Messpunkt (P) als proportional zu c.φ/(2.π.f.) /Δt ermittelt wird,

wobei c die Geschwindigkeit der Ultraschallwellen in der Luft angibt, f die Frequenz der Ultraschallwellen angibt und Δt ein Zeitintervall zwischen den beiden Messschritten ist.

22. Vorrichtung zum Erkennen von Bewegungen einer Ultraschallwellen reflektierenden Oberfläche (21), mit:

einer Ultraschallwellenemissionsvorrichtung (2),
einer Ultraschallwellenempfangsvorrichtung (3),
einer Steuervorrichtung (5), welche die Ultraschallwellenemissionsvorrichtung (2) steuert und von der Ultraschallwellenempfangsvorrichtung (3) erfasste Signale empfängt, wobei
die Steuervorrichtung (5) dazu ausgebildet ist, mehrere aufeinanderfolgende Messschritte durchzuführen, bei welchen die Ultraschallwellenemissionsvorrichtung (2) mindestens eine einfallende Ultraschallwelle in Richtung der Oberfläche (21) in die Luft emittiert und die Ultraschallwellenempfangsvorrichtung (3) reflektierte Signale, welche ausgehend von der mindestens einen einfallenden Ultraschallwelle mindestens eine von der Oberfläche (21) in die Luft reflektierte Ultraschallwelle repräsentieren, erfasst,
die Ultraschallwellenempfangsvorrichtung (3) ein Netz von Empfangswandlern (3) mit mehreren Ultraschallempfangswandler (3a) ist,
wobei die Vorrichtung **dadurch gekennzeichnet ist, dass** die Ultraschallwellenemissionsvorrichtung (2) dazu ausgebildet ist, mehrere mindestens zur Oberfläche (21) gehörende Messpunkte (P) mit der mindestens einen einfallenden Ultraschallwelle unter mehreren Einfallswinkeln zu bestrahlen,
dass die Steuervorrichtung (5) dazu ausgebildet ist, in jedem Messschritt ein Kanalbildungssignal für jeden Messpunkt (P) durch Bilden zumindest von Empfangskanälen anhand der reflektierten Signale zu ermitteln,
und dass die Steuervorrichtung (5) dazu ausgebildet ist, die Bewegungen der Oberfläche (21) am betrachteten Messpunkt (P) zu ermitteln, indem mindestens eine Verzögerung oder eine Phasenverschiebung zwischen zwei Kanalbildungssignalen für diesen Messpunkt (P) ermittelt wird.

**Claims**

1. A method for detecting movements of a surface (21) reflecting ultrasonic waves, comprising several successive measuring steps during each of which at least one incident ultrasonic wave is emitted into the air towards the surface (21) using an ultrasonic wave emitting device (2) and reflected signals representative of at least one ultrasonic wave reflected in the air by said surface (21) from said at least one incident ultrasonic wave are detected, **characterized in that,** during each measuring step:

   - the movements of a plurality of measuring points (P) belonging at least to said surface are measured by illuminating each measuring point (P) with said at least one incident ultrasonic wave at a multiplicity of angles of incidence,
   - the reflected signals are detected using a network of receiving transducers (3) comprising a plurality of ultrasonic receiving transducers (3a) and a beam-forming signal is determined for each measuring point (P), by at least beam-forming in reception from said reflected signals, **and in that** it further comprises at least one movement determination step during which said movements of the surface (21) at the considered measuring point (P) are determined by determining at least one delay or phase shift between two beam-forming signals for this measuring point (P).

2. The method according to claim 1, wherein during each measuring step, each measuring point (P) of the surface (21) is illuminated with said at least one incident wave at angles of incidence extending over a range of angles of incidence of at least 20 degrees.

3. The method according to claim 1 or claim 2, wherein, during each measuring step, the movements are measured at substantially any point of the surface (21) over an area greater than 10 cm$^2$.

4. The method according to any one of the preceding claims, wherein the ultrasonic wave emitting device (2) and the network of receiving transducers (3) are two-dimensional.

5. The method according to claim 4, wherein the network of emitting transducers (2) has an aperture ($OE_Y$, $OE_Z$, $OE'_Y$, $OE'_Z$) at least equal to an aperture ($OR_{Y'}$, $OR_Z$) of the ultrasonic wave receiving device (3) in two substantially perpendicular directions.

6. The method according to claim 5, wherein the aperture ($OE_Y$, $OE_Z$, $OE'_Y$, $OE'_Z$) of the network of emitting transducers (2) is equal to at least three times the aperture ($OR_Y$, $OR_Z$) of the ultrasonic wave receiving device (3), in at least one of the directions.

7. The method according to any one of claims 4 to 6, wherein the aperture ($OE_Y$, $OE_Z$, $OE'_Y$, $OE'_Z$) of the ultrasonic wave emitting device (2) is at least equal to 20 cm in each direction.

8. The method according to any one of the preceding claims, wherein the ultrasonic wave emitting device (2) comprises a network of emitting transducers (2) comprising a plurality of ultrasonic emitting transducers (2a).

9. The method according to claim 8, wherein the ultrasonic emitting transducers (2a) are divided into several groups and during said measuring step, a same signal is simultaneously emitted by the ultrasonic emitting transducers (2a) belonging to a same group.

10. The method according to any one of the preceding claims, wherein the ultrasonic wave emitting device (2) comprises at least one ultrasonic emitting transducer (2a) so arranged as to emit into a mixing cavity (4) configured to cause multiple reflections of said at least one incident ultrasonic wave before sending it towards the surface (21).

11. The method according to any one of the preceding claims, wherein the ultrasonic waves have a frequency of less than 100 kHz.

12. The method according to any one of the preceding claims, wherein the ultrasonic waves are emitted at a rate above 500 shots per second.

13. The method according to any one of the preceding claims, wherein, during each step of measuring index k, a beam-forming signal $S_k$ in reception is calculated at said point (P), at least at different points (P) of the surface (21), and in each movement determination step, the movement of each point (P) of the surface (21) is determined by determining a delay or phase shift between the beam-forming signals $S_k$ in reception at said point (P), for two different k values.

14. The method according to claim 13, wherein during each step of measuring index k, the ultrasonic wave emitting device (2) emits a non focused incident ultrasonic wave towards the surface (21).

15. The method according to claim 13, wherein, during each step of measuring index k, the ultrasonic wave emitting device (2) successively emits focused incident ultrasonic waves towards the different points (P) of the surface (21) and the beam-forming signal $S_k$ in reception at said point (P) is determined from the reflected signals corresponding to the focused incident ultrasonic wave at said point (P).

16. The method according to any one of claims 13 to 15, wherein the beam-forming signal $S_k$ in reception is determined by the following formula:

$$S_k(t) = \sum_{j=1}^{N} r_j \left( t - \frac{d_j}{C} \right)$$

where:

- $r_j$ is the signal detected by the ultrasonic receiving transducer (3a) of index j,
- t is time,
- $d_j$ is a distance between the point P and the ultrasonic receiving transducer (3a) of index j,
- c is the speed of the ultrasonic wave in the air.

17. The method according to any one of claims 1 to 12, wherein the ultrasonic wave emitting device (2) comprises a network of emitting transducers (2) comprising a plurality of ultrasonic emitting transducers (2a), and wherein during each k-index measuring step, respective impulse responses are measured between each ultrasonic emitting transducer (2a) and each ultrasonic receiving transducer (3a), and then at least at different points (P) of the surface (21) a beam-forming signal $S'_k$ in emission and in reception is calculated at said point (P), and during each movement determination step, a movement of each point (P) of the surface (21) is determined by determining a delay or phase shift between the beam-forming signals $S'_k$ in emission and in reception at said point (P), for two different k values.

18. The method according to claim 17, wherein each beam-forming signal in emission and in reception at said point (P) is determined by the following formula:

$$S'_k(t) = \sum_{j=1}^{N} \sum_{i=1}^{M} h_{ijk}\left(t - \frac{d_{ij}}{c}\right) \ ,$$

where:

- i is an index between 1 and M referring to an ultrasonic emitting transducer (2a),
- j is an index between 1 and N referring to an ultrasonic receiving transducer (3a),
- $h_{ijk}$ is the impulse response between the ultrasonic emitting transducer (2a) of index i and the ultrasonic receiving transducer (3a) of index j,
- t is time,
- $d_{ij}$ is a distance travelled by an ultrasonic wave from the ultrasonic emitting transducer (2a) of index i to the ultrasonic receiving transducer (3a) of index j by reflecting at the considered point (P) of the surface (21),
- c is the speed of the ultrasonic waves in the air.

19. The method according to any one of the preceding claims, wherein, during at least some measuring steps, a beam-forming signal is determined for each measuring point (P) of a predetermined observation area and the measuring points (P) belonging to the surface (21) are determined as those which maximize the beam-forming signal.

20. The method according to any one of the preceding claims, wherein, during the movement determination step, a delay dt between beam-forming signals corresponding to two measuring steps at the same measuring point (P) is determined and:

- a travel $\delta$ at the measuring point (P) as being proportional to dt.c
- and/or a speed at the measuring point (P) as being proportional to dt.c/$\Delta$t,

where c is the speed of the ultrasonic waves in the air and $\Delta$t is a time interval between said two measuring steps.

21. The method according to any one of claims 1 to 19, wherein, during the movement determination step, a phase shift $\varphi$ is determined between beam-forming signals corresponding to two measuring steps at the same measuring point (P) and:

- a travel $\delta$ at the measuring point (P) as being proportional to c.$\varphi$/(2.$\pi$.f)
- and/or a speed at the measuring point (P) as being proportional to c.$\varphi$/(2.$\pi$.f)/$\Delta$t,

where c is the speed of the ultrasonic waves in the air, f is the frequency of the ultrasonic waves and $\Delta$t is a time interval between said two measuring steps.

22. A device for detecting movements of a surface (21) reflecting ultrasonic waves, comprising

an ultrasonic wave emitting device (2),
an ultrasonic wave receiving device (3),
a control device (5) controlling the ultrasonic wave emitting device (2) and receiving signals detected by the ultrasonic wave receiving device (3), wherein
the control device (5) is configured to carry out several successive measuring steps during each of which the ultrasonic wave emitting device (2) emits at least one incident ultrasonic wave into the air towards the surface (21) and the ultrasonic wave receiving device (3) receives reflected signals representative of at least one ultrasonic wave reflected in the air by said surface (21) from said at least one incident ultrasonic wave,
the ultrasonic wave receiving device (3) is a network of receiving transducers (3) comprising a plurality of receiving ultrasonic transducers (3a),

**the device being characterized in that** the ultrasonic wave emitting device (2) is configured to illuminate a plurality of measuring points (P) belonging at least to said surface (21) by said at least one incident ultrasonic wave at a multiplicity of angles of incidence,
**in that** the control device (5) is configured to determine, during each measuring step, a beam-forming signal for each measuring point (P), by at least beam-forming in reception from said reflected signals,
**and in that** the control device (5) is configured to determine said movements of the surface (21) at the considered measuring point (P) by determining at least one delay or phase shift between two beam-forming signals for that

measuring point (P).

**FIG. 1**

FIG. 2

FIG. 3

FIG. 4

OE'Y

15

4

18

OE'Z

17

16

20

13

OEZ

2a

2

OEY

19

3

ORZ

3a

ORY'

FIG. 5

FIG. 6

FIG. 7

V (mm/s)

FIG. 8

D (μm)

FIG. 9

EP 3 488 261 B1

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 4122427 A

- US 2011208060 A

**Littérature non-brevet citée dans la description**

- **NICOLAS QUIEFFIN ; STEFAN CATHELINE ; ROS KIRI ING ; MATHIAS FINK.** Acoustic source localization model using in-skull reverberation and time reversal. *Applied Physics Letters,* 2007, vol. 90, 063902

- **N. ETAIX ; M. FINK ; R. K. ING.** Acoustic imaging device with one transducer. *J. Acoust. Soc. Am.,* 2012, vol. 131 (5), EL395-EL399
- **N. ETAIX ; J. DUBOIS ; M. FINK ; R. K. ING.** Increasing the modal density in plates for mono-element focusing in air. *J. Acoust. Soc. Am.,* 2013, vol. 134 (2), 1049-1054